# EUROPEAN PATENT APPLICATION

(11) **EP 1 908 492 A1**
(43) Date of publication of application: **09.04.2008**
(21) Application number: 06767132.1
(22) Date of filing: 22.06.2006
(51) Int. Cl.: A61P 25/28

(54) **METABOTROPIC GLUTAMATE RECEPTOR ACTIVATOR**

(30) Priority: 22.06.2005 JP 2005182046
(71) Applicant: Ajinomoto Co., Inc., Tokyo 104-8315 (JP)
(72) Inventor: TAKESHITA, Sen AJINOMOTO CO., INC., Kanagawa 2108681 (JP); OHSU, Takeaki c/o AJINOMOTO CO., INC., Kawasaki-shi Kanagawa 2108681 (JP); ETO, Yuzuru c/o AJINOMOTO CO., INC., Kawasaki-shi Kanagawa 2108681 (JP); TAKAHASHI, Mitsuo c/o AJINOMOTO CO., INC., Kawasaki-shi Kanagawa 2108681 (JP)
(74) Representative: Nicholls, Kathryn Margaret
(86) International application number: PCT/JP2006/312474
(87) International publication number: WO 2006/137469

(57) **Abstract**

Amino acids other than glutamic acid are used as a metabotropic glutamate receptor activator. More preferably, aspartic acid, valine and cysteine are used as a group I metabotropic glutamate receptor activator; alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine and hydroxyproline are used as a group II metabotropic glutamate receptor activator; and cysteine is used as a group III metabotropic glutamate receptor activator.

## Description

### Technical Field

The present invention relates to a metabotropic glutamate receptor activator comprising an amino acid other than glutamic acid. The present invention also relates to a metabotropic glutamate receptor activator comprising an amino acid other than glutamic acid and a nucleic acid and/or a cation. The present invention further relates to a metabotropic glutamate receptor activator comprising glutamic acid and a nucleic acid, or glutamic acid, a nucleic acid, and a cation.

### Background Art

Glutamic acid, which is one of amino acids, is a major excitatory neurotransmitter in a central nervous system of mammals. Electrophysiological, anatomical, and biochemical analyses suggest that glutamic acid is involved in many neuron action processes such as fast excitatory synaptic transmission, control of neurotransmitter release, long-term potentiation, learning/memory, incremental synaptic plasticity, hypoxic-ischemic injury/death of nerve cell, epileptiform seizure, and lesions of some neurodegenerative disorders.
Moreover, the recent report revealed that glutamic acid has ability not only to act as a neurotransmitter in the central nervous system but also, when glutamic acid acts on a digestive tract such as stomach or intestine, ability to promote secretion of gastric acid or movement of digestive tract. It is recognized that glutamic acid acts effectively not only in nerve cells but also in the whole body.

In recent years, studies on glutamate receptors have been made to clarify action mechanism of glutamic acid (Non-patent Documents 1 to 8). The glutamate receptors are classified into ionotropic receptors and metabotropic receptors. Ionotropic glutamate receptors are ligand-gated ion channel, and cations enter in a cell by the action of glutamic acid. On the other hand, metabotropic glutamate receptors are G-protein-coupled receptor, and an extracellular signal is converted into an intracellular second messenger by the action of glutamic acid depending on the kinds of G-proteins.

The metabotropic glutamate receptors include eight subtypes (mGluR1 to mGluR8), and are classified into three groups (groups I, II, and III) based on the homology of amino acid sequences. The homology of amino acid sequences in each group is conserved (about 70%), but the homology among different groups is 50% or less. Some of the activators and inhibitors of metabotropic glutamate receptors reported so far are specific in each subtype, but many of the activators and inhibitors are common to each group, and therefore the structures of the receptors of each group are expected to be similar to each other.

The receptors of each group binds to the same kind of G-proteins. Metabotropic glutamate receptors belonging to the group I are subtypes 1 and 5 (mGluR1 and mGluR5), and when glutamic acid acts on these receptors, these receptors are coupled to Gq to activate phospholipase C, resulting in production of IP3. Metabotropic glutamate receptors belonging to the group II are subtypes 2 and 3 (mGluR2 and mGluR3), and when glutamic acid acts on these receptors, these receptors are coupled to Gi to inhibit adenylate cyclase, resulting in a decrease in intracellular cAMP. Metabotropic glutamate receptors belonging to the group III are subtypes 4, 6, 7 and 8 (mGluR4, mGluR6, mGluR7, and mGluR8), and when glutamic acid acts on these receptors, these receptors are coupled to Gi to inhibit adenylate cyclase, resulting in a decrease in intracellular cAMP.

The metabotropic glutamate receptors may act to promote or inhibit functions in a living body. For neurological diseases, liver diseases, cardiovascular diseases, gastrointestinal diseases, and other diseases, therapeutic agents having activation effects and therapeutic agents having inhibition effects may be selected depending on clinical conditions.
Functional analyses of metabotropic glutamate receptors have been made mainly on nerve cells, and therefore application studies have been made mainly on neurological diseases up to the present. However, the results of gene expression analyses or the like revealed that metabotropic glutamate receptors are widely distributed in the whole body other than the central nervous system and suggested that the receptors may be involved in various biological functions or causes of diseases. For example, it has been reported that: group I metabotropic glutamate receptors are expressed in liver, heart, and lymphocytes; group II metabotropic glutamate receptors are expressed in heart and small intestine; and group III metabotropic glutamate receptors are expressed in pancreas; and these receptors are involved in cell functions. The inventors of the present invention have confirmed that these receptors are expressed in various tissues in a living body by RT-PCR analysis using RNAs extracted from tissues of human and rat. Therefore, it is not too much to say that application values of activators and inhibitors of metabotropic glutamate receptors are being rapidly enhanced.
Non-patent Document 1: J Pharmacol Exp Ther. 2003 Apr;305(1):131-42
Non-patent Document 2: Epilepsia. 2003 Sep;44(9):1153-9
Non-patent Document 3: Chem Senses. 2000 Oct;25(5):507-15.
Non-patent Document 4: Ann N Y Acad sci. 1998 Nov 30;855:398-406.
Non-patent Document 5: Physiol Behav. 1991 May;49(5):843-54.
Non-patent Document 6: J Neurosci Res. 2004 Feb 15;75(4):472-9.
Non-patent Document 7: FEBS Lett. 2003 Jun 19;545(2-3):233-8.
Non-patent Document 8: Science. 1998 Mar 13; 279(5357):1722-5.

### Disclosure of the Invention

Many specific activators have been developed as metabotropic glutamate receptor activator, but only a few of them are compounds present in a living body, such as glutamic acid and cysteic acid. Therefore, therapeutic agents for various diseases that contain those activators have major problems of side effects or permeability.
Among amino acids, only L-glutamic acid has been known as an activator of a metabotropic glutamate receptor, and no activator has been developed based on the amino acids other than L-glutamic acid. Meanwhile, an animal experiment using mice or rats has revealed that a nerve action potential induced by L-glutamic acid is enhanced by a nucleotide such as guanylic acid and inosinic acid, but in the experiment, a specific biomolecule induced by L-glutamic acid remains a matter of speculation. Although a metal divalent ion such as calcium or magnesium is known to cause a response singly or together with L-glutamic acid with respect to a metabotropic glutamate receptor, only L-glutamic acid was used as an amino acid in the experiment, and it is unknown whether a similar effect is obtained in the case of another amino acid. Amino acids other than glutamic acid are not known to be useful as metabotropic glutamate receptor activators.
Moreover, it has not been known that activation of metabotropic glutamate receptors is enhanced by a nucleic acid and a cation.
That is, it is an object of the present invention to provide a novel metabotropic glutamate receptor activator that is highly safe for a living body.

The inventors of the present invention revealed that amino acids other than glutamic acid can activate metabotropic glutamate receptors. In addition, they have further revealed that activation of the receptor by amino acids other than glutamic acid is enhanced by coexistence with a nucleic acid, in particular, a nucleotide such as inosinic acid or guanylic acid, and a cation, in particular, a metal divalent ion such as calcium or magnesium. Moreover, the inventors have found that activation of metabotropic glutamate receptors by glutamic acid is also enhanced by the nucleic acid, or the nucleic acid and cation. Based on such findings, they have completed the present invention.

That is, the present invention is as follows.
(1) A metabotropic glutamate receptor activator comprising one or more kinds of amino acids selected from amino acids other than glutamic acid.
(2) The metabotropic glutamate receptor activator according to (1), further comprising a nucleic acid.
(3) The metabotropic glutamate receptor activator according to (1) or (2), further comprising a cation.
(4) A group I metabotropic glutamate receptor activator comprising one or more kinds of amino acids selected from the group consisting of aspartic acid, valine, and cysteine.
(5) The group I metabotropic glutamate receptor activator according to (4), further comprising a nucleic acid.
(6) The group I metabotropic glutamate receptor activator according to (4) or (5), further comprising a cation.
(7) A group II metabotropic glutamate receptor activator comprising one or more kinds of amino acids selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, and hydroxyproline.
(8) The group II metabotropic glutamate receptor activator according to (7), further comprising a nucleic acid.
(9) The group II metabotropic glutamate receptor activator according to (7) or (8), further comprising a cation.
(10) A group III metabotropic glutamate receptor activator comprising cysteine.
(11) The group III metabotropic glutamate receptor activator according to (10), further comprising a nucleic acid.
(12) The group III metabotropic glutamate receptor activator according to (10) or (11), further comprising a cation.
(13) A medicament comprising as an active ingredient the metabotropic glutamate receptor activator according to any one of (1) to (12), for preventing or treating any of internal diseases, neurological diseases, cardiovascular diseases, surgical diseases, neurosurgical diseases, thoracic surgical diseases, orthopedic diseases, obstetric and gynecologic diseases, urological diseases, pediatric diseases, ophthalmic diseases, otorhinolaryngologic diseases, dermatologic diseases, and dental diseases.
(14) A method of screening an inhibitor for metabotropic glutamate receptor activation, comprising using an amino acid other than glutamic acid.
(15) The method according to (14), wherein the metabotropic glutamate receptor is a group I metabotropic glutamate receptor, and the amino acid other than glutamic acid is aspartic acid, valine, or cysteine.
(16) The method according to (14), wherein the metabotropic glutamate receptor is a group II metabotropic glutamate receptor, and the amino acid other than glutamic acid is alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, or hydroxyproline.
(17) The method according to (14), wherein the metabotropic glutamate receptor is a group III metabotropic glutamate receptor, and the amino acid other than the glutamic acid is cysteine.
(18) A metabotropic glutamate receptor activator comprising (i) glutamic acid and a nucleic acid or (ii) glutamic acid, a nucleic acid, and a cation.
(19) A group I metabotropic glutamate receptor activator comprising (i) glutamic acid and a nucleic acid or (ii) glutamic acid, a nucleic acid, and a cation.
(20) A group II metabotropic glutamate receptor activator comprising (i) glutamic acid and a nucleic acid or (ii) glutamic acid, a nucleic acid, and a cation.
(21) A group III metabotropic glutamate receptor activator comprising (i) glutamic acid and a nucleic acid or (ii) glutamic acid, a nucleic acid, and a cation.
(22) A medicament comprising as an active ingredient the activator according to any one of (18) to (21), for preventing or treating any of internal diseases, neurological diseases, cardiovascular diseases, surgical diseases, neurosurgical diseases, thoracic surgical diseases, orthopedic diseases, obstetric and gynecologic diseases, urological diseases, pediatric diseases, ophthalmic diseases, otorhinolaryngologic diseases, dermatologic diseases, and dental diseases.
(23) A method of screening an inhibitor for a metabotropic glutamate receptor activation, comprising using (i) glutamic acid and a nucleic acid, or (ii) glutamic acid, a nucleic acid, and a cation.
(24) The method according to (23), wherein the metabotropic glutamate receptor is a group I metabotropic glutamate receptor, a group II metabotropic glutamate receptor or a group III metabotropic glutamate receptor.

### Brief Description of the Drawings

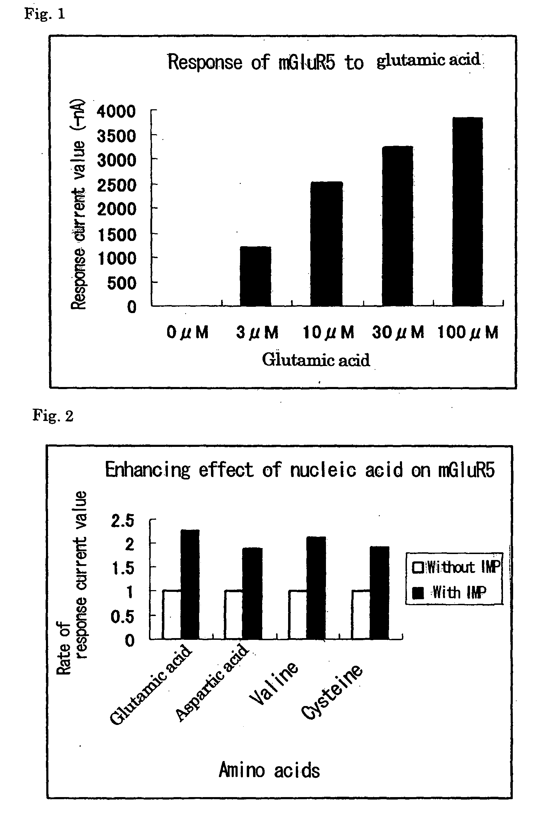

Fig. 1 is a graph showing an effect of glutamic acid on a group I metabotropic glutamate receptor. cRNA of mGluR5 was microinjected in *Xenopus* oocytes, followed by culture at 18°C for 3 days. Intracellular currents were recorded at the time of addition of any concentration of glutamic acid. The maximum value of the intracellular currents was defined as a response current value.
Fig. 2 is a graph showing effects of amino acids and a nucleic acid on a group I metabotropic glutamate receptor. cRNA of mGluR5 was microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a nucleic acid (IMP). This graph shows ratios of the response current values at the time of addition of both an amino acid and the nucleic acid (IMP) to the response current values at the time of addition of only an amino acid (defined as 1).

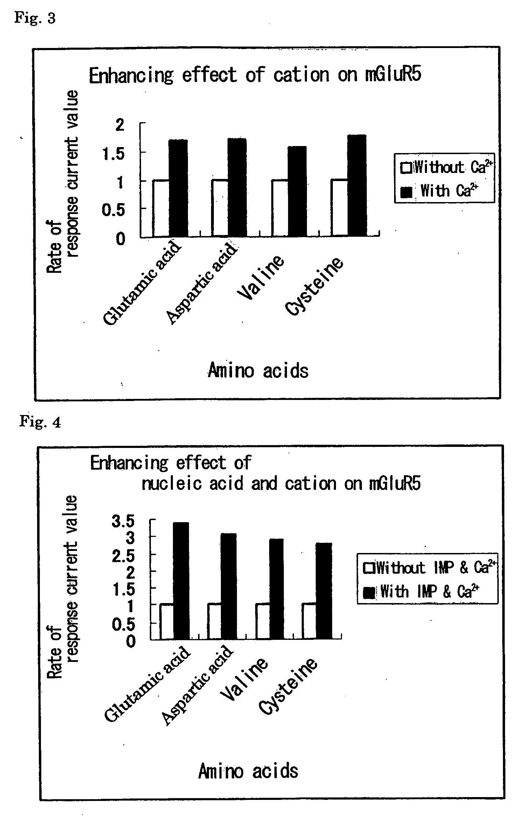

Fig. 3 is a graph showing effects of amino acids and a cation on a group I metabotropic glutamate receptor. cRNA of mGluR5 was microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a cation (Ca²⁺). This graph shows ratios of the response current values at the time of addition of both an amino acid and the cation (Ca²⁺) to the response current values at the time of addition of only an amino acid (defined as 1).
Fig. 4 is a graph showing effects of amino acids, a nucleic acid, and a cation on a group I metabotropic glutamate receptor. cRNA of mGluR5 was microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a nucleic acid (IMP) and a cation (Ca²⁺). This graph shows ratios of the response current values at the time of addition of an amino acid, the nucleic acid (IMP), and the cation (Ca²⁺) to the response current values at the time of addition of only an amino acid (defined as 1).

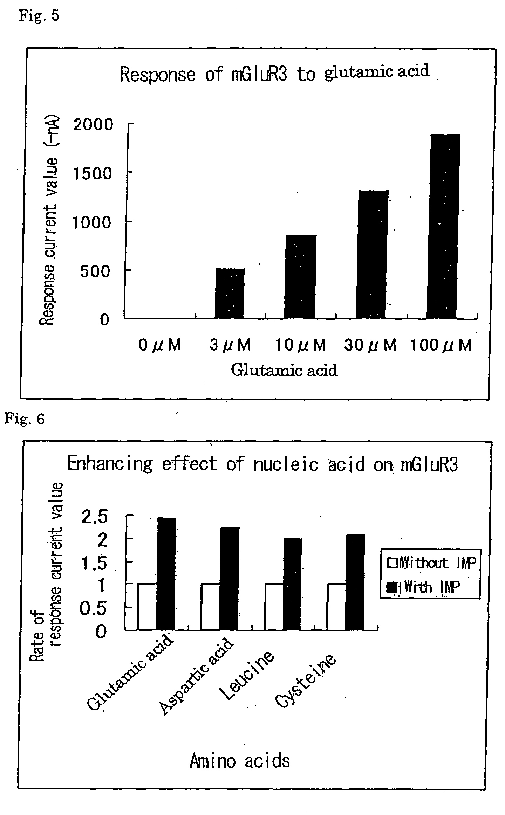

Fig. 5 is a graph showing an effect of glutamic acid on a group II metabotropic glutamate receptor. cRNAs of mGluR3, GIRK1, and GIRK4 were microinjected in *Xenopus* oocytes, followed by culture at 18°C for 3 days. Intracellular currents were recorded at the time of addition of any concentration of glutamic acid. The maximum value of the intracellular currents was defined as a response current value.
Fig. 6 is a graph showing effects of amino acids and a nucleic acid on a group II metabotropic glutamate receptor. cRNAs of mGluR3, GIRK1, and GIRK4 were microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a nucleic acid (IMP). This graph shows ratios of the response current values at the time of addition of both an amino acid and the nucleic acid (IMP) to the response current values at the time of addition of only an amino acid (defined as 1).

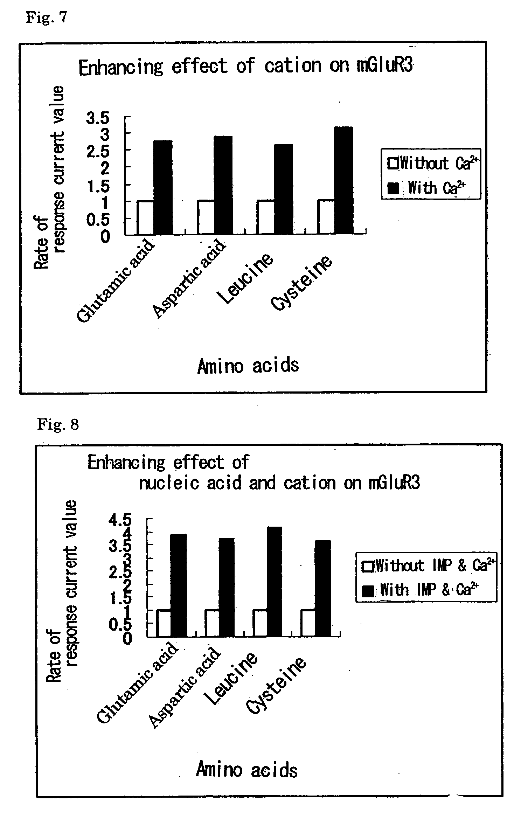

Fig. 7 is a graph showing effects of amino acids and a cation on a group II metabotropic glutamate receptor. cRNAs of mGluR3, GIRK1, and GIRK4 were microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a cation (Ca²⁺). This graph shows ratios of the response current values at the time of addition of both an amino acid and the cation (Ca²⁺) to the response current values at the time of addition of only an amino acid (defined as 1).
Fig. 8 is a graph showing effects of amino acids, a nucleic acid, and a cation on a group II metabotropic glutamate receptor. cRNAs of mGluR3, GIRK1, and GIRK4 were microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a nucleic acid (IMP) and a cation (Ca²⁺). This graph shows ratios of the response current values at the time of addition of an amino acid, the nucleic acid (IMP), and the cation (Ca²⁺) to the response current values at the time of addition of only an amino acid (defined as 1).

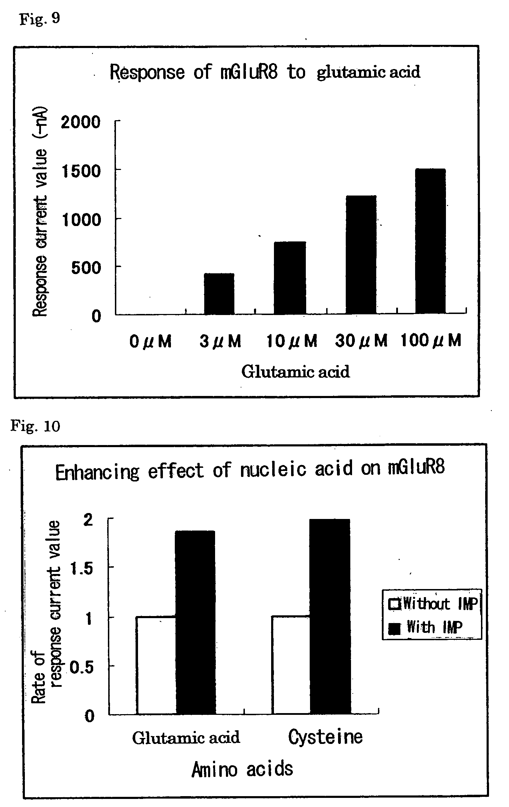

Fig. 9 is a graph showing effects of glutamic acid on a group III metabotropic glutamate receptor. cRNAs of mGluR8, GIRK1, and GIRK4 were microinjected in *Xenopus* oocytes, followed by culture at 18°C for 3 days. Intracellular currents were recorded at the time of addition of any concentration of glutamic acid. The maximum value of the intracellular currents was defined as a response current value.
Fig. 10 is a graph showing effects of amino acids and a nucleic acid on a group III metabotropic glutamate receptor. cRNAs of mGluR8, GIRK1, and GIRK4 were microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a nucleic acid (IMP). This graph shows ratios of the response current values at the time of addition of both an amino acid and the nucleic acid (IMP) to the response current values at the time of addition of only an amino acid (defined as 1).

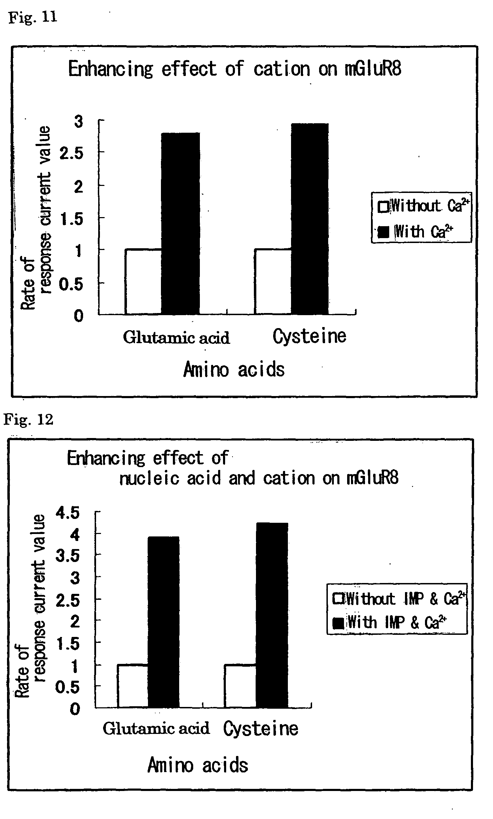

Fig. 11 is a graph showing effects of amino acids and a cation on a group III metabotropic glutamate receptor. cRNAs of mGluR8, GIRK1, and GIRK4 were microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a cation (Ca²⁺). This graph shows ratios of the response current values at the time of addition of both an amino acid and the cation (Ca²⁺) to the response current values at the time of addition of only an amino acid (defined as 1).
Fig. 12 is a graph showing effects of amino acids, a nucleic acid, and a cation on a group III metabotropic glutamate receptor. cRNAs of mGluR8, GIRK1, and GIRK4 were microinjected in *Xenopus* oocytes, followed by culture at 18°C for 3 days. Intracellular currents were recorded at the time of addition of any concentration of each amino acid. Intracellular currents were recorded at the time of addition of the same concentration of each amino acid together with a nucleic acid (IMP) and a cation (Ca²⁺). This graph shows ratios of the response current values at the time of addition of an amino acid, the nucleic acid (IMP), and the cation (Ca²⁺) to the response current values at the time of addition of only an amino acid (defined as 1).

### Description of the Preferred Embodiments

Hereinafter, the present invention will be described. In the specification of the present invention, the respective amino acids mean L-amino acids unless otherwise specified.
A metabotropic glutamate receptor activator of the present invention comprises one or more kinds of amino acids selected from amino acids other than glutamic acid. The "amino acids other than glutamic acid" include 19 kinds of amino acids (other than glutamic acid) out of 20 kinds of amino acids that make up proteins, and ornithine, taurine, and hydroxyproline.
The metabotropic glutamate receptor activator of the present invention may comprise one or more kinds of amino acids of the "amino acids other than glutamic acid" and may contain glutamic acid as additional component.

Aspartic acid, valine, and cysteine can activate a metabotropic glutamate receptor belonging to the group I. Therefore, one or more kinds of amino acids selected from aspartic acid, valine, and cysteine can be used as activators for a metabotropic glutamate receptor belonging to the group I.
Examples of the metabotropic glutamate receptor belonging to the group I include a metabotropic glutamate receptor subtype 1 (mGluR1) and a metabotropic glutamate receptor subtype 5 (mGluR5). Examples of the mGluR1 include mGluR1 encoded by human mGluR1 gene that is registered in the GenBank under Accession No. NM#000838.
Examples of the mGluR5 include mGluR5 encoded by human mGluR5 gene that is registered in the GenBank under Accession No. NM#000842. The mGluR1 or mGluR5 is not limited to a protein encoded by a gene having the above-mentioned sequence and may be a protein encoded by a gene having homology of 60% or more, preferably 80% or more, more preferably 90% or more, particularly preferably 95% or more to the above-mentioned sequence as long as it encodes a protein having function as the receptor. The functions of the receptors can be examined by expressing these genes in cells and measuring changes in currents or changes in concentrations of calcium ion at the time of adding glutamic acid.

Alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, and hydroxyproline activate the metabotropic glutamate receptor belonging to group II. Therefore, one or more kinds of amino acids selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, and hydroxyproline can be used as an activator for the metabotropic glutamate receptor belonging to group II.
Examples of the metabotropic glutamate receptor belonging to the group II include a metabotropic glutamate receptor subtype 2 (mGluR2) and a metabotropic glutamate receptor subtype 3 (mGluR3). An example of the mGluR2 includes mGluR2 encoded by human mGluR2 gene that is registered in GenBank under Accession No. NM#000839. An example of the mGluR3 includes mGluR3 encoded by human mGluR3 gene that is registered in GenBank under Accession No. NM#000840. The mGluR2 or mGluR3 is not limited to a protein encoded by a gene having the above-mentioned sequence and may be a protein encoded by a gene having homology of 60% or more, preferably 80% or more, more preferably 90% or more, particularly preferably 95% or more to the above-mentioned sequence as long as it encodes a protein having function of the receptor.

Cysteine activates a metabotropic glutamate receptor belonging to the group III. Therefore, cysteine can be used as an agonist for a metabotropic glutamate receptor belonging to the group III.
Examples of the metabotropic glutamate receptor belonging to the group III include a metabotropic glutamate receptor subtype 4 (mGluR4), a metabotropic glutamate receptor subtype 6 (mGluR6), a metabotropic glutamate receptor subtype 7 (mGluR7), and a metabotropic glutamate receptor subtype 8 (mGluR8). An example of the mGluR4 includes mGluR4 encoded by human mGluR4 gene that is registered in GenBank under Accession No. NM#000841. An example of the mGluR6 includes mGluR6 encoded by human mGluR6 gene that is registered in GenBank under Accession No. NM#000843. An example of the mGluR7 includes mGluR7 encoded by human mGluR7 gene that is registered in GenBank under Accession No. NM#000844. An example of the mGluR8 includes mGluR8 encoded by human mGluR8 gene that is registered in GenBank under Accession No. NM#000845.
The mGluR4, mGluR6, mGluR7, or mGluR8 is not limited to a protein encoded by a gene having the above-mentioned sequence and may be a protein encoded by a gene having homology of 60% or more, preferably 80% or more, more preferably 90% or more, particularly preferably 95% or more to the above-mentioned sequence as long as it encodes a protein having function of the receptor.

The metabotropic glutamate receptor activator of the present invention may be an activator not only for the above-mentioned human metabotropic glutamate receptors but also for metabotropic glutamate receptors of mammals such as mice, rats, and dogs.

Activation of metabotropic glutamate receptors by the above-mentioned amino acids other than glutamic acid can be confirmed by using living cells where the metabotropic glutamate receptor or a fragment thereof is expressed, a cell membrane where the metabotropic glutamate receptor or a fragment thereof is expressed, an in vitro system including a protein of the metabotropic glutamate receptor or a fragment thereof, etc.
Hereinafter, a method of confirming the activation using living cells will be shown as an example, but the method is not limited to the example.
One or a plurality of eight subtypes of metabotropic glutamate receptors are expressed in culture cells such as *Xenopus* oocytes, hamster ovary cells, or human embryonic renal cells. This can be achieved by introducing one or more of eight metabotropic glutamate receptor genes cloned in plasmids into cells as they are or as cRNAs obtained by using the plasmids as templates. A metabotropic glutamate receptor activation reaction can be detected by an electrophysiological method or by using a fluorescent indicator for elevated intracellular calcium. The functions of group II and group III metabotropic glutamate receptors are analyzed preferably by using a coupled G-protein or the like together with the receptor, and for example, it is possible to use a method of simultaneously expressing G-protein α15 or α16, or a chimeric G protein of Gq and Gi and the receptor, or in the case of *Xenopus* oocytes, a method of simultaneously expressing G-protein-gated inward rectifier K channel (GIRK) and the receptor.

First, the expression of a metabotropic glutamate receptor is confirmed by a response caused by L-glutamic acid or a specific activator. Oocytes where an intracellular current (nA) was caused, or culture cells where fluorescence of a fluorescent indicator was caused by about 10 µM L-glutamic acid are used. The concentration dependency is determined with varying concentrations of L-glutamic acid. Subsequently, solutions of about 1 µM to 100 mM amino acids other than L-glutamic acid are prepared and added to the oocytes or culture cells, to thereby determine whether each amino acid acts as an activator for the metabotropic glutamate receptor.

The metabotropic glutamate receptor activator of the present invention may comprise a nucleic acid in addition to the above-mentioned amino acids other than glutamic acid. That is, the present invention provides: (1) a group I metabotropic glutamate receptor activator comprising one or more kinds of amino acids selected from aspartic acid, valine, and cysteine, and a nucleic acid; (2) a group II metabotropic glutamate receptor activator comprising one or more kinds of amino acids selected from alanine, arginine, asparagine, aspartic acid, cysteine, glycine, histidine, isoleucine, leucine, lysine, methionine, phenylalanine, proline, serine, threonine, tryptophan, tyrosine, valine, ornithine, taurine, and hydroxyproline, and a nucleic acid; and (3) a group III metabotropic glutamate receptor activator comprising cysteine and a nucleic acid.
Herein, the nucleic acid is preferably a nucleotide such as inosinic acid or guanylic acid, more preferably inosine monophosphate.
When a metabotropic glutamate receptor activator comprises a nucleic acid, a receptor is more strongly activated. Alternatively, in cells where a receptor is not activated by only an amino acid, the receptor is activated by the coexistence with a nucleic acid at the same concentration of the amino acid.
The ratio of an amino acid and a nucleic acid in a metabotropic glutamate receptor activator of the present invention is not particularly limited as long as the receptor can be activated, but the weight ratio of a nucleic acid and an amino acid is preferably 1:10 to 10:1.

The metabotropic glutamate receptor activator of the present invention may contain a cation in addition to the amino acids other than glutamic acid. That is, the present invention provides: (1) a group I metabotropic glutamate receptor activator comprising one or more kinds of amino acids selected from aspartic acid, valine, and cysteine, and a cation; (2) a group II metabotropic glutamate receptor activator comprising one or more kinds of amino acids selected from alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, and hydroxyproline, and a cation; and (3) a group III metabotropic glutamate receptor activator comprising cysteine and a cation.
Herein, the cation is preferably a metal cation, more preferably bivalent metal cations such as calcium ion and magnesium ion, and particularly preferably calcium ion.
When a metabotropic glutamate receptor activator comprises a cation, a receptor is more strongly activated. Alternatively, in cells where a receptor is not activated by only an amino acid, the receptor is activated by the coexistence with a cation at the same concentration of the amino acid.
The ratio of an amino acid and a cation in a metabotropic glutamate receptor activator of the present invention is not particularly limited as long as the receptor can be activated, but the weight ratio of a cation and an amino acid is preferably 1:10 to 10:1.

The inventors of the present invention have discovered that activation of a group I metabotropic glutamate receptor, a group II metabotropic glutamate receptor, or a group III metabotropic glutamate receptor by glutamic acid is enhanced with a nucleic acid, or a nucleic acid and a cation.
Therefore, the metabotropic glutamate receptor activator of the present invention may be a group I metabotropic glutamate receptor activator, a group II metabotropic glutamate receptor activator, or a group III metabotropic glutamate receptor activator comprising (i) glutamic acid and a nucleic acid, or (ii) glutamic acid, a nucleic acid, and a cation.

A metabotropic glutamate receptor is expressed in various tissues and plays a role in various physiological functions. Meanwhile, activators for a metabotropic glutamate receptor have been developed as therapeutic or preventive agents for many diseases such as internal diseases and nervous system diseases (for example, Ann NY Acad Sci. 2003 Nov; 1003:12-21.; Annu Rev Pharmacol Toxicol. 1997; 37:205-37.; J Med Chem. 2000 Jul 13; 43(14):2609-45, etc.).
Therefore, the metabotropic glutamate receptor activator of the present invention can be used as an active ingredient of a medicament for preventing or treating a disease associated with a metabotropic glutamate receptor. Examples of the disease associated with a metabotropic glutamate receptor include internal diseases, neurological diseases, cardiovascular diseases, surgical diseases, neurosurgical diseases, thoracic surgical diseases, orthopedic diseases, obstetric and gynecologic diseases, urological diseases, pediatric diseases, ophthalmic diseases, otorhinolaryngologic diseases, dermatologic diseases, and dental diseases.

A metabotropic glutamate receptor activator according to the present invention can be applied as a medicament by oral administration, invasive administration using an injection or the like, suppository administration, or transdermal administration. A mixture of an active ingredient and a solid or liquid nontoxic carrier for medicament suitable for an administration method such as oral administration or injection may be administered in the form of a conventional pharmaceutical preparation. Examples of the form include: solid forms such as tablet, granule, powder, and capsule; liquid forms such as solution, suspension, and emulsion; and another form such as freeze-dried preparation. These preparations can be prepared by conventional pharmaceutical methods. Moreover, if necessary, conventional additives such as a stabilizer, wetting agent, emulsifier, binder, and tonicity-adjusting agent may be appropriately added.
The dose of a drug of the present invention may be an amount effective for treatment or prevention, and is appropriately adjusted depending on patient's age, sex, body weight, symptom, etc., and for example, the amount of an amino acid per kg body weight at an administration is preferably 0.01 g to 10 g, more preferably 0.1 g to 1 g per kg body weight. The number of administration is not particularly limited, and the medicament may be administered once or several times a day.

The metabotropic glutamate receptor activator of the present invention may also be used as a food or drink effective for treatment or prevention a disease associated with a metabotropic glutamate receptor. For example, the activator can be provided as a food or drink supplied in a container or package representing its effect on treatment or prevention of a disease associated with a metabotropic glutamate receptor as described above.

The present invention also provides a method of screening an inhibitor of a metabotropic glutamate receptor activation characterized by using an amino acid other than glutamic acid.
For example, a compound to inhibit the activation of a metabotropic glutamate receptor belonging to the group I (group I metabotropic glutamate receptor antagonist) can be screened by: adding aspartic acid, valine, or cysteine, and a test compound to *Xenopus* oocytes or mammal-derived culture cells where a group I metabotropic glutamate receptor is expressed; measuring intercellular current values or intercellular calcium contents; and selecting a compound which inhibits an increase in a current value or calcium content caused by aspartic acid, valine, or cysteine.
A compound to inhibit the activation of a metabotropic glutamate receptor belonging to the group II (group II metabotropic glutamate receptor antagonist) can be screened by: adding alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, or hydroxyproline, and a test compound to cells where a group II metabotropic glutamate receptor is expressed; measuring intercellular current values or intercellular calcium contents; and selecting a compound which inhibits an increase in a current value or intercellular calcium content caused by these amino acids.
A compound to inhibit the activation of a metabotropic glutamate receptor belonging to the group III (group III metabotropic glutamate receptor antagonist) can be screened by: adding cysteine and a test compound to cells where a group III metabotropic glutamate receptor is expressed; measuring an intracellular current value or intercellular calcium content; and selecting a compound which inhibits an increase in a current value or intercellular calcium content caused by cysteine.
The above-mentioned screening systems may contain a nucleic acid or a cation in addition to the above-mentioned amino acids.
The test compounds to be used for the screening may be low-molecular-weight compounds, saccharides, peptides, proteins, etc.

Moreover, a method of screening an inhibitor of a metabotropic glutamate receptor activation of the present invention may be a method of screening a substance to inhibit the receptor activation by using glutamic acid and a nucleic acid, or by using glutamic acid, a nucleic acid, and a cation.

### EXAMPLES

Hereinafter, the present invention will be described in more detail, but the scope of the present invention is not limited to these Examples.

### Example 1

### Preparation of genes (cRNA)

A metabotropic glutamate receptor subtype 5 (mGluR5) was used as a group I metabotropic glutamate receptor. A metabotropic glutamate receptor subtype 3 (mGluR3) was used as a representative of group II metabotropic glutamate receptors. A metabotropic glutamate receptor subtype 8 (mGluR8) was used as a representative of group III metabotropic glutamate receptors. The genes of the three metabotropic glutamate receptors, G-protein-gated inward rectifier K channel 1 (GIRK1), and G-protein-gated inward rectifier K channel 4 (GIRK4) were prepared as follows. Synthetic oligo-DNAs for PCR (forward primer (N) and reverse primer (C)) were designed based on DNA sequences registered in the NCBI (National Center for Biotechnology Information) (mGluR5: NM #000842, mGluR3: NM #000840, mGluR8: NM #000845, GIRK1: NM #002239, GIRK4: NM #002239) (Table 1) (SEQ ID NOS: 1-10).

**[Table 1]**

| Name | Sequence (5'-3') |
|---|---|
| mGluR5-N | ACTAATACGACTCACTATAGGGCCTAAAATGGTCCTTCTGTTG |
| mGluR5-C | TTCACAACGACGAGGAGCT |
| mGluR3-N | ACTAATACGACTCACTATAGGGAAGATGTTGACAAGACTGCAAG |
| mGluR3-C | ATCACAGAGATGAGGTGGTGG |
| mGluR8-N | ACTAATACGACTCACTATAGGGAAAATGGTATGCGAGGGAAAG |
| mGluR8-C | TTCAGATTGAATGATTGCTGTAAC |
| GIRK1-N | ACTAATACGACTCACTATAGGGATGTCTGCACTCCGAAGGA |
| GIRK1-C | TTATGTGAAGCGATCAGAGTTCA |
| GIRK4-N | ACTAATACGACTCACTATAGGGATGGCTGGCGATTCTAGGA |
| GIRK4-C | AGGAGGTCTTAGGGAGGCTG |

PCR was performed using a cDNA derived from the human brain (manufactured by Clontech) as a material and using Pfu ultra DNA Polymerase (manufactured by Stratagene) under the following conditions. After a 3-minute reaction at 94°C, a cycle of 94°C/30 seconds, 55°C/30 seconds, and 72°C/2 minutes was repeated 35 times, followed by a 7-minute reaction at 72°C. Amplified PCR products were subjected to agarose gel electrophoresis, stained with a DNA staining reagent and detected by UV irradiation. The lengths of the PCR products were confirmed by comparing the lengths with a DNA marker having a known size, which was electrophoresed simultaneously with the PCR products. A plasmid vector pBR322 was cleaved with a restriction enzyme EcoRV (manufactured by TAKARA BIO INC.). The respective gene fragments amplified by PCR were ligated to the cleaved site using Ligation Kit (manufactured by Promega). *Escherichia coli* DH5α strain was transformed with the reaction solution, and transformants carrying plasmids where the PCR products were cloned were selected. The PCR amplified products were identified by DNA sequence analysis. The recombinant plasmids were used as a template to prepare cRNA of each of the receptor genes using a cRNA preparation kit (Ambion, Inc.).

### Example 2

### Preparation of amino acid, nucleic acid, and cation

Special grade reagents of twenty-three L-amino acids including alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, ornithine, taurine, and hydroxyproline were used. Special grade reagents of inosine and calcium chloride were used. Glutamine and cysteine were prepared every time before use, and the other reagents were prepared and stored at -20°C. A solution for dissolving amino acids, nucleic acids, and cations, a solution for preparing *Xenopus* oocytes, and a solution for culturing *Xenopus* oocytes have the following composition. NaCl 96 mM / KCl 2 mM / MgCl₂ 1 mM / CaCl₂ 1.8 mM / Hepes 5 mM / pH 7.2.

### Example 3

### Electrophysiological measurement method using Xenopus oocyte

Ca ion concentration-dependent Cl ion current measurement method using a *Xenopus* oocyte expression system was used as a method of measuring the signaling via a metabotropic glutamate receptor. The abdomen of a *Xenopus* were incised, and the egg mass was removed and treated with a 1 % collagenase solution at 20°C for 2 hours, to thereby obtain individual oocytes. To one oocyte was introduced 50 nl of 1 µg/µl receptor cRNA or 50 nl of distilled water using a microcapillary, followed by culture at 18°C for 2 or 3 days. This culture was performed using a solution obtained by adding 2 mM pyruvic acid, 10 U/ml penicillin, and 10 µg/ml streptomycin to the solution shown in Example 2. After completion of culture, a ligand solution was added to oocytes injected with the cRNA and oocytes injected with distilled water, respectively. Electrophysiological measurement was performed using an amplifier Geneclamp 500 (manufactured by Axon Instruments, Inc.) and recording software AxoScope 9.0 (manufactured by Axon Instruments, Inc.). The oocytes were subjected to membrane potential fixation at -70 mV by a two-electrode membrane potential fixation method, and intracellular currents via Ca ion concentration-dependent Cl ion were measured. The maximal value of the intracellular currents was defined as a response current value.

### Example 4

### Effect of glutamic acid on group I metabotropic glutamate receptor

The effect of glutamic acid on a group I metabotropic glutamate receptor was studied by the method described in Example 3. Fig. 1 shows the results of reactions caused by glutamic acid (3 µM, 10 µM, 30 µM, 100 µM) in oocytes injected with the cRNA of a group I metabotropic glutamate receptor subtype 5 (mGluR5) or in oocytes injected with distilled water. The results revealed that the cRNA of the metabotropic glutamate receptor injected in the oocytes was functionally expressed. Meanwhile, the oocytes injected with water did not react to glutamic acid at high concentration, and it was found that the metabotropic glutamate receptor was not expressed in the oocytes themselves.

### Example 5

### Effect of amino acids on group I metabotropic glutamate receptor

The effects of amino acids other than glutamic acid on a group I metabotropic glutamate receptor were studied by the method described in Example 3. Table 2 shows the results of reactions caused by alanine (10 mM), arginine (10 mM), asparagine (10 mM), aspartic acid (300 µM), cysteine (1 mM), glutamine (10 mM), glycine (10 mM), histidine (10 mM), isoleucine (10 mM), leucine (10 mM), lysine (10 mM), methionine (10 mM), phenylalanine (10 mM), proline (10 mM), serine (10 mM), threonine (10 mM), tryptophan (10 mM), tyrosine (10 mM), valine (3 mM), ornithine (10 mM), taurine (10 mM), and hydroxyproline (10 mM) in oocytes injected with the cRNA of a group I metabotropic glutamate receptor subtype 5 (mGluR5) or in oocytes injected with distilled water. The results revealed that not only glutamic acid but also aspartic acid, cysteine, and valine have effects of enhancing the group I metabotropic glutamate receptor.

**[Table 2]**

| | mGluR5 | mgluR3 | mgluR8 |
|---|---|---|---|
| Alanine | | Activated | |
| Arginine | | Activated | |
| Asparagine | | Activated | |
| Aspartic acid | Activated | Activated | |
| Cysteine | Activated | Activated | Activated |
| Glutamine | | Activated | |
| Glutamic acid | Activated | Activated | Activated |
| Glycine | | Activated | |
| Histidine | | Activated | |
| Isoleucine | | Activated | |
| Leucine | | Activated | |
| Lysine | | Activated | |
| Methionine | | Activated | |
| Phenylalanine | | Activated | |
| Proline | | Activated | |
| Serine | | Activated | |
| Threonine | | Activated | |
| Tryptophan | | Activated | |
| Tyrosine | | Activated | |
| Valine | Activated | Activated | |
| Ornithine | | Activated | |
| Taurine | | Activated | |
| Hydroxyproline | | Activated | |

### Example 6

### Effect of amino acid and nucleic acid on group I metabotropic glutamate receptor

The effects of amino acids including glutamic acid and a nucleic acid on a group I metabotropic glutamate receptor were studied by the method described in Example 3. Fig. 2 shows the results indicating that the effects of glutamic acid (3 µM), aspartic acid (300 µM), valine (3 mM), and cysteine (3 mM) were enhanced by the coexistence with inosinic acid (1 mM) in oocytes injected with the cRNA of a group I metabotropic glutamate receptor subtype 5 (mGluR5) or distilled water. The results revealed that response current values were increased by the coexistence with the nucleic acid even at the same concentration of the amino acids. In addition, even under a low concentration of an amino acid which induces no response current, a response current was detected by the coexistence with a nucleic acid.

### Example 7

### Effect of amino acid and cation on group I metabotropic glutamate receptor

The effects of amino acids including glutamic acid and a cation on a group I metabotropic glutamate receptor were studied by the method described in Example 3. Fig. 3 shows the results indicating that the effects of glutamic acid (3 µM), aspartic acid (300 µM), valine (3 mM), and cysteine (3 mM) were enhanced by the coexistence with calcium ion (10 mM) in oocytes injected with the cRNA of a group I metabotropic glutamate receptor subtype 5 (mGluR5) or distilled water. The results revealed that response current values were increased by the coexistence with the cation even at the same concentration of the amino acids. In addition, even under a low concentration of an amino acid which induces no response current, a response current was detected by the coexistence with a cation.

### Example 8

### Effect of amino acid, nucleic acid, and cation on group I metabotropic glutamate receptor

The effects of amino acids including glutamic acid, a nucleic acid, and a cation on a group I metabotropic glutamate receptor were studied by the method described in Example 3. Fig. 4 shows the results indicating that the effects of glutamic acid (3 µM), aspartic acid (300 µM), valine (3 mM), and cysteine (1 mM) were enhanced by the coexistence with both of inosinic acid (1 mM) and calcium ion (10 mM) in oocytes injected with the cRNA of a group I metabotropic glutamate receptor subtype 5 (mGluR5) or in oocytes injected with distilled water. The results revealed that response current values were increased by the coexistence with the cation even at the same concentration of the amino acids. In addition, even in a solution containing either the nucleic acid or the cation and containing the amino acid at the same concentration, which induces no response current, a response current was detected by supplementing either cation or nucleic acid.

### Example 9

### Effect of glutamic acid on group II metabotropic glutamate receptor

The effect of glutamic acid on a group II metabotropic glutamate receptor was studied by the method described in Example 3. Fig. 5 shows the results of reactions caused group II metabotropic glutamate receptor subtype 3 (mGluR3) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that the cRNA of the metabotropic glutamate receptor injected in the oocytes was functionally expressed. Meanwhile, the oocytes injected with water did not react to glutamic acid at high concentration, and thus it was found that the metabotropic glutamate receptor was not expressed in the oocytes themselves.

### Example 10

### Effect of amino acid on group II metabotropic glutamate receptor

The effects of amino acids on a group II metabotropic glutamate receptor were studied by the method described in Example 3. Table 2 shows the results of reactions caused by 1 mM alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, and hydroxyproline in oocytes injected with the cRNA of a group II metabotropic glutamate receptor subtype 3 (mGluR3) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that not only glutamic acid but also alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, and hydroxyproline have effects of enhancing the activation of the group II metabotropic glutamate receptor.

### Example 11

### Effect of amino acid and nucleic acid on group II metabotropic glutamate receptor

The effects of amino acids including glutamic acid and a nucleic acid on a group II metabotropic glutamate receptor were studied by the method described in Example 3. Fig. 6 shows the results indicating that the effects of glutamic acid (3 µM), aspartic acid (300 µM), valine (1 mM), and leucine (1 mM), were enhanced by the coexistence with inosinic acid (1 mM) in oocytes injected with the cRNA of a group II metabotropic glutamate receptor subtype 3 (mGluR3), G-protein-gated inward rectifier K channel 1 (GIRK1), and water. The results revealed that response current values were increased by the coexistence with the cation even at the same concentration of the amino acids. In addition, even at an amino acid concentration at which response current was not induced, a response current was detected by the coexistence with the nucleic acid. Similar effects of the nucleic acid was observed on 4 amino acids arbitrarily selected from the 23 amino acids having effects on a group II metabotropic glutamate receptor shown in Example 10, so the effects of the nucleic acid were suggested to be universal for the amino acids.

### Example 12

### Effect of amino acid and cation on group II metabotropic glutamate receptor

The effects of amino acids other than glutamic acid and a cation on a group II metabotropic glutamate receptor were studied by the method described in Example 3. Fig. 7 shows the results indicating that the effects of glutamic acid (3 µM), aspartic acid (300 µM), leucine (1 mM), and cysteine (1 mM) were enhanced by the coexistence with calcium ion (10 mM) in oocytes injected with the cRNA of a group II metabotropic glutamate receptor subtype 3 (mGluR3) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that response current values were increased by the coexistence with the cation at the same concentration of the amino acids. In addition, even at an amino acid concentration at which response current was not induced, a response current was detected by the coexistence with the cation. Similar effects of the cation was observed on 4 amino acids arbitrarily selected from the 23 amino acids having effects on a group II metabotropic glutamate receptor shown in Example 10, so the effects of the cation were suggested to be universal for the amino acids.

### Example 13

### Effect of amino acid, nucleic acid, and cation on group II metabotropic glutamate receptor

The effects of amino acids including glutamic acid, nucleic acid, and a cation on a group II metabotropic glutamate receptor were studied by the method described in Example 3. Fig. 8 shows the results indicating that the effects of glutamic acid (3 µM), aspartic acid (300 µM), leucine (1 mM), and cysteine (1 mM) were enhanced by the coexistence with inosinic metabotropic glutamate receptor subtype 3 (mGluR3) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that response current values were increased by the coexistence with the nucleic acid and the cation even at the same concentration of the amino acids. In addition, even at an amino acid concentration at which response current was not induced, a response current was detected by the coexistence with the nucleic acid and the cation. Similar effects of the cation was observed on 4 amino acids arbitrarily selected from the 23 amino acids having effects on a group II metabotropic glutamate receptor shown in Example 10, so the effects of the nucleic acid and the cation were suggested to be universal for the amino acids.

### Example 14

### Effect of glutamic acid on group III metabotropic glutamate receptor

The effect of glutamic acid on a group III metabotropic glutamate receptor was studied by the method described in Example 3. Fig. 9 shows the results of reactions caused by glutamic acid (3 µM, 10 µM, 30 µM, 100 µM) in oocytes injected with the cRNA of a group II metabotropic glutamate receptor subtype 8 (mGluR8) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that the cRNA of the metabotropic glutamate receptor injected in the oocytes was functionally expressed. Meanwhile, the oocytes injected with water did not react to glutamic acid at high concentration, and it was found that the metabotropic glutamate receptor was not expressed in the oocytes themselves.

### Example 15

### Effect of amino acid on group III metabotropic glutamate receptor

The effects of amino acids other than glutamic acid on a group III metabotropic glutamate receptor were studied by the method described in Example 3. Table 2 shows the results of reactions caused by alanine (10 mM), arginine (10 mM), asparagine (10 mM), aspartic acid (300 µM), cysteine (1 mM), glutamine (10 mM), glycine (10 mM), histidine (10 mM), isoleucine (10 mM), leucine (10 mM), lysine (10 mM), methionine (10 mM), phenylalanine (10 mM), proline (10 mM), serine (10 mM), threonine (10 mM), tryptophan (10 mM), tyrosine (10 mM), valine (3 mM), ornithine (10 mM), taurine (10 mM), and hydroxyproline (10 mM) in oocytes injected with the cRNAofa group III metabotropic glutamate receptor subtype 8 (mGluR8) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that not only glutamic acid but also cysteine have effects of activating the group III metabotropic glutamate receptor.

### Example 16

### Effect of cysteine and nucleic acid on group III metabotropic glutamate receptor

The effects of amino acids including glutamic acid, a nucleic acid, and a cation on a group III metabotropic glutamate receptor were studied by the method described in Example 3. Fig. 10 shows the results indicating that the effects of glutamic acid (3 µM) and cysteine (1 mM) were enhanced by the coexistence with inosinic acid (1 mM) in oocytes injected with the cRNA of a group III metabotropic glutamate receptor subtype 8 (mGluR8) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that response current values were increased by the coexistence with the nucleic acid at the same concentration of cysteine. In addition, even at a cysteine concentration at which response current was not induced, a response current was detected by the coexistence with the nucleic acid.

### Example 17

### Effect of cysteine and cation on group III metabotropic glutamate receptor

The effects of amino acids including glutamic acid and a cation on a group III metabotropic glutamate receptor were studied by the method described in Example 3. Fig. 11 shows the results indicating that the effects of glutamic acid (3 µM) and cysteine (1 mM) were enhanced by the coexistence with calcium ion (10 mM) in oocytes injected with the cRNA of a group III metabotropic glutamate receptor subtype 8 (mGluR8) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that response current values were increased by the coexistence with the cation at the same concentration of cysteine. In addition, even at a cysteine concentration at which response current was not induced, a response current was detected by the coexistence with the cation.

### Example 18

### Effect of cysteine, nucleic acid, and cation on group III metabotropic glutamate receptor

The effects of amino acids including glutamic acid, a nucleic acid, and a cation on a group I metabotropic glutamate receptor were studied by the method described in Example 3. Fig. 12 shows the results indicating that the effects of glutamic acid (3 µM) and cysteine (1 mM) were enhanced by the coexistence with both of inosinic acid (1 mM) and calcium ion (10 mM) in oocytes injected with the cRNA of a group III metabotropic glutamate receptor subtype 8 (mGluR8) together with the cRNAs of G-protein-gated inward rectifier K channel 1 (GIRK1) and G-protein-gated inward rectifier K channel 4 (GIRK4) (2.5 ng each) or in oocytes injected with distilled water as a control. The results revealed that response current values were increased by the coexistence with the cation at the same concentration of cysteine. In addition, even in a solution containing either the nucleic acid or the cation and containing the same concentration of cysteine by which response current was not induced, a response current was detected by supplementing either cation or nucleic acid.

It is well known that receptor structures and receptor properties are similar in each group of metabotropic glutamate receptors belonging to any of groups I, II, and III, and activators and inhibitors common to each group can be used. The typical examples thereof are as follows.
Group I metabotropic glutamate receptor activator: DHPG
Group I metabotropic glutamate receptor inhibitor: AIDA
Group II metabotropic glutamate receptor activator: DCG-IV
Group II metabotropic glutamate receptor inhibitor: LY341195
Group III metabotropic glutamate receptor activator: L-AP4
Group III metabotropic glutamate receptor inhibitor: CPPG
Reference: Psychopharmacology Springer-Verlag 2005, [Review; Ionotropic and metabotropic glutamate receptor structure and pharmacology] James N.C.Kew and John A.Kemp
Therefore, receptor activation experiments were performed in this example using mGluR5 as a group I metabotropic glutamate receptor, mGluR3 as a group II metabotropic glutamate receptor, and mGluR8 as a group III metabotropic glutamate receptor, but other metabotropic glutamate receptors belonging to the respective groups were considered to be activated by the above-mentioned amino acids other than glutamic acid.

### Example 19

### Distribution of metabotropic glutamate receptor in a living body

To clarify the distribution of metabotropic glutamate receptors in a living body, the tissue expressions of metabotropic glutamate receptors subtypes 1 to 8 were examined using RNA obtained by a rat as a material by RT-PCR in accordance with the following procedures. Preparation of RNAs from rat tissues was performed as follows. The cerebrum, cerebellum, lung, heart, liver, kidney, adrenal gland, thyroid gland, parathyroid gland, pancreas, spleen, esophagus, upper part of stomach, fundus of stomach, duodenum, jejunum, ileum, cecum, colon, rectum, testis, epididymis, bladder, bone marrow, gastrocnemial muscle, soleus muscle, skeletal muscle, fat, prostate gland, tongue, sublingual gland, and thymus gland were isolated from a 15-week-old male F344 rat. Preparation of the total RNAs was performed using Isogen (manufactured by Nippon Gene Co., Ltd.). The tissues were homogenized using Fast Prep (BIO 101) or Polytron homogenizer, and the total RNAs were extracted from the homogenates. The cDNAs were synthesized using the Total RNAs as templates and using OligodT primer and Superscript III reverse transcriptase (manufactured by Invitrogen). RT-PCR for the metabotropic glutamate receptors subtypes 1 to 8 were performed using the synthetic primers shown in Table 3 (SEQ ID NOS: 11 to 26) in accordance with the following procedures. The expression levels of metabotropic glutamate receptor genes were analyzed by quantitative PCR using the cDNAs of the respective tissues as templates and using SYBR Green Realtime PCR Master Mix (manufactured by TOYOBO Co., Ltd.) and ABI PRISM 7700 Sequence Detector. The tissue distribution of expression is shown in Table 4. The results revealed that the metabotropic glutamate receptors were expressed not only in the brain but also in the whole body, which suggested that the metabotropic glutamate receptors were involved not only in the central functions but also in various physiological functions of peripheral tissues and peripheral organs.

**[Table 3]**

| Name | Sequence (5'-3') |
|---|---|
| GRM1-f | TATCTGAGTCGGTCCTCTGCAC |
| GRM1-r | ACACACTACAGGGTGGAAGAGC |
| GRM2-f | CAAGTGCCCGGAGAACTTCAAC |
| GRM2-r | CACTGGAGGTGACATAGAAGATAG |
| GRM3-f | CAACGAAGCATGCCTATGC |
| GRM3-r | ACTGTAACCTTACCCTGTA |
| GRM4-f | GCTACCAAACAGACCTACG |
| GRM4-r | CCAATGTGCCATCCTCAGA |
| GRM5-f | CCCCAAACTCTCCAGTCTC. |
| GRM5-r | TCACAACGATGAAGAACTC |
| GRM6-f | CCAAACCACCACACTAACTGTGTC |
| GRM6-r | GGATGGAACAGGATGACGTAGG |
| GRM7-f | TCTAACCTGTTCCATGCCA |
| GRM7-r | ATAGGAAGCGGGCAGGAC |
| GRM8-f | CGCAAGAGAAGCTTCAAGGCTG |
| GRM8-r | TCACCTCGCCATTTGGTCTGTC |

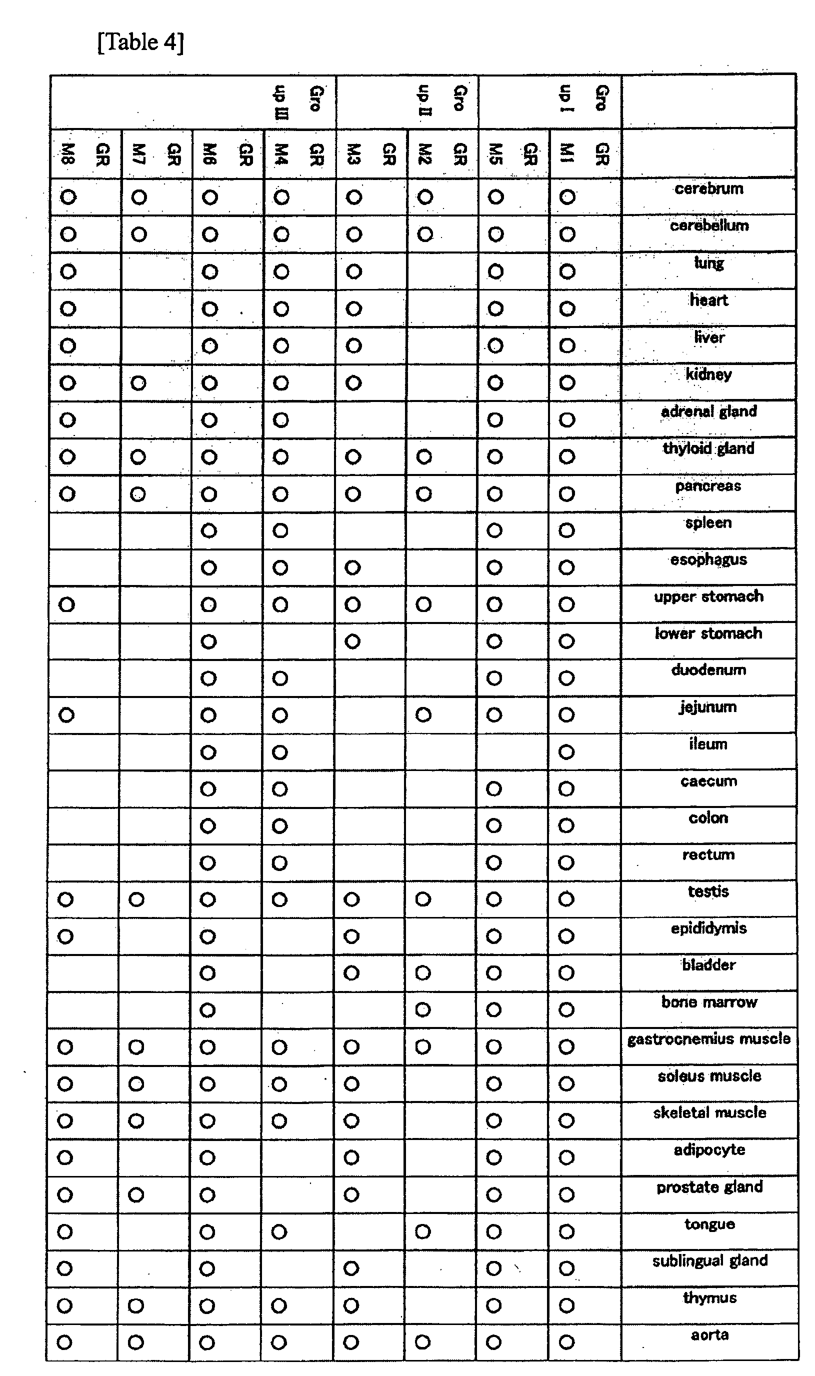

### Industrial Applicability

Activators for GPCRs (G-protein coupled receptors) can be universally applied as therapeutic agents for various diseases. Typical examples of the therapeutic agents that have already been in the market or are now being developed include an acetylcholine receptor activator (for treating Alzheimer's disease), an adrenaline receptor activator (for treating bronchial asthma or obesity), an opioid receptor activator (for treating pain), a receptor activator (for treating pain), a calcitonin receptor activator (for treating osteoporosis), a cholecystokinin receptor activator (for treating diabetes), a serotinin receptor activator (for treating migraine), a dopamine receptor activator (for treating Parkinson's disease, sedative), a melatonin receptor activator (for treating primary insomnia), and a melanocortin receptor activator (for treating obesity).
As shown in Example 19 and Table 3, the respective subtypes of metabotropic glutamate receptors are distributed in various tissues, so it is predicted that the activators for metabotropic glutamate receptors belonging to the GPCRs can be applied to various diseases as therapeutic agents. In addition, metabotropic glutamate receptors are distributed also in the tongue or digestive tract, so the activators can be applied not only as therapeutic agents but also in the food field.

## Claims

1. A metabotropic glutamate receptor activator comprising one or more kinds of amino acids selected from amino acids other than glutamic acid.

2. The metabotropic glutamate receptor activator according to claim 1, further comprising a nucleic acid.

3. The metabotropic glutamate receptor activator according to claim 1 or 2, further comprising a cation.

4. A group I metabotropic glutamate receptor activator comprising one or more kinds of amino acids selected from the group consisting of aspartic acid, valine, and cysteine.

5. The group I metabotropic glutamate receptor activator according to claim 4, further comprising a nucleic acid.

6. The group I metabotropic glutamate receptor activator according to claim 4 or 5, further comprising a cation.

7. A group II metabotropic glutamate receptor activator comprising one or more kinds of amino acids selected from the group consisting of alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, and hydroxyproline.

8. The group II metabotropic glutamate receptor activator according to claim 7, further comprising a nucleic acid.

9. The group II metabotropic glutamate receptor activator according to claim 7 or 8, further comprising a cation.

10. A group III metabotropic glutamate receptor activator comprising cysteine.

11. The group III metabotropic glutamate receptor activator according to claim 10, further comprising a nucleic acid.

12. The group III metabotropic glutamate receptor activator according to claim 10 or 11, further comprising a cation.

13. A medicament comprising as an active ingredient the metabotropic glutamate receptor activator according to any one of claims 1 to 12, for preventing or treating any of internal diseases, neurological diseases, cardiovascular diseases, surgical diseases, neurosurgical diseases, thoracic surgical diseases, orthopedic diseases, obstetric and gynecologic diseases, urological diseases, pediatric diseases, ophthalmic diseases, otorhinolaryngologic diseases, dermatologic diseases, and dental diseases.

14. A method of screening an inhibitor for metabotropic glutamate receptor activation, comprising using an amino acid other than glutamic acid.

15. The method according to claim 14, wherein the metabotropic glutamate receptor is a group I metabotropic glutamate receptor, and the amino acid other than glutamic acid is aspartic acid, valine, or cysteine.

16. The method according to claim 14, wherein the metabotropic glutamate receptor is a group II metabotropic glutamate receptor, and the amino acid other than glutamic acid is alanine, arginine, asparagine, aspartic acid, cysteine, glutamine, glycine, histidine, isoleucine, leucine, lysine, methionine, phenylalanine, proline, serine, threonine, tryptophan, tyrosine, valine, ornithine, taurine, or hydroxyproline.

17. The method according to claim 14, wherein the metabotropic glutamate receptor is a group III metabotropic glutamate receptor, and the amino acid other than the glutamic acid is cysteine.

18. A metabotropic glutamate receptor activator comprising (i) glutamic acid and a nucleic acid or (ii) glutamic acid, a nucleic acid, and a cation.

19. A group I metabotropic glutamate receptor activator comprising (i) glutamic acid and a nucleic acid or (ii) glutamic acid, a nucleic acid, and a cation.

20. A group II metabotropic glutamate receptor activator comprising (i) glutamic acid and a nucleic acid or (ii) glutamic acid, a nucleic acid, and a cation.

21. A group III metabotropic glutamate receptor activator comprising (i) glutamic acid and a nucleic acid or (ii) glutamic acid, a nucleic acid, and a cation.

22. A medicament comprising as an active ingredient the activator according to any one of claims 18 to 21, for preventing or treating any of internal diseases, neurological diseases, cardiovascular diseases, surgical diseases, neurosurgical diseases, thoracic surgical diseases, orthopedic diseases, obstetric and gynecologic diseases, urological diseases, pediatric diseases, ophthalmic diseases, otorhinolaryngologic diseases, dermatologic diseases, and dental diseases.

23. A method of screening an inhibitor for a metabotropic glutamate receptor activation, comprising using (i) glutamic acid and a nucleic acid, or (ii) glutamic acid, a nucleic acid, and a cation.

24. The method according to claim 23, wherein the metabotropic glutamate receptor is a group I metabotropic glutamate receptor, a group II metabotropic glutamate receptor or a group III metabotropic glutamate receptor.


SEQUENCE LISTING

<110> Ajinomoto Co., Inc.

<120> METABOTROPIC GLUTAMATE RECEPTOR ACTIVATOR

<130> C593-C6066

<150> JP2005-182046
<151> 2005-06-22

<160> 26

<170> PatentIn version 3.1

<210> 1
<211> 43
<212> DNA
<213> Artificial sequence

<220>
<223> mGluR5-N primer

<400> 1
actaatacga ctcactatag ggcctaaaat ggtccttctg ttg 43

<210> 2
<211> 19
<212> DNA
<213> Artificial sequence

<220>
<223> mGluR5-C primer

<400> 2
ttcacaacga cgaggagct 19

<210> 3
<211> 44
<212> DNA
<213> Artificial sequence

<220>

<223>  mGluR3-N primer

<400>  3
actaatacga ctcactatag ggaagatgtt gacaagactg caag                44

<210>  4
<211>  21
<212>  DNA
<213>  Artificial sequence

<220>
<223>  mGluR3-C primer

<400>  4
atcacagaga tgaggtggtg g                                          21

<210>  5
<211>  43
<212>  DNA
<213>  Artificial sequence

<220>
<223>  mGluR8-N primer

<400>  5
actaatacga ctcactatag ggaaaatggt atgcgaggga aag                  43

<210>  6
<211>  24
<212>  DNA
<213>  Artificial sequence

<220>
<223>  mGluR8-C primer

<400>  6
ttcagattga atgattgctg taac                                       24

<210>  7
<211>  41
<212>  DNA

<213>  Artificial sequence

<220>
<223>  GIRK1-N primer

<400>  7
actaatacga ctcactatag ggatgtctgc actccgaagg a                41

<210>  8
<211>  23
<212>  DNA
<213>  Artificial sequence

<220>
<223>  GIRK1-C primer

<400>  8
ttatgtgaag cgatcagagt tca                                    23

<210>  9
<211>  41
<212>  DNA
<213>  Artificial sequence

<220>
<223>  GIRK4-N primer

<400>  9
actaatacga ctcactatag ggatggctgg cgattctagg a                41

<210>  10
<211>  20
<212>  DNA
<213>  Artificial sequence

<220>
<223>  GIRK4-C primer

<400>  10
aggaggtctt agggaggctg                                        20

<210> 11
<211> 22
<212> DNA
<213> Artificial sequence

<220>
<223> GRM1 forward primer

<400> 11
tatctgagtc ggtcctctgc ac 22

<210> 12
<211> 22
<212> DNA
<213> Artificial sequence

<220>
<223> GRM1 reverse primer

<400> 12
acacactaca gggtggaaga gc 22

<210> 13
<211> 22
<212> DNA
<213> Artificial sequence

<220>
<223> GRM2 forward primer

<400> 13
caagtgcccg gagaacttca ac 22

<210> 14
<211> 24
<212> DNA
<213> Artificial sequence

<220>
<223> GRM2 reverse primer

<400> 14

cactggaggt gacatagaag atag 24

<210> 15
<211> 19
<212> DNA
<213> Artificial sequence

<220>
<223> GRM3 forward primer

<400> 15
caacgaagca tgcctatgc 19

<210> 16
<211> 19
<212> DNA
<213> Artificial sequence

<220>
<223> GRM3 reverse primer

<400> 16
actgtaacct taccctgta 19

<210> 17
<211> 19
<212> DNA
<213> Artificial sequence

<220>
<223> GRM4 forward primer

<400> 17
gctaccaaac agacctacg 19

<210> 18
<211> 19
<212> DNA
<213> Artificial sequence

<220>

<223>  GRM4 reverse primer

<400>  18
ccaatgtgcc atcctcaga                                                    19



<210>  19
<211>  19
<212>  DNA
<213>  Artificial sequence

<220>
<223>  GRM5 forward primer

<400>  19
ccccaaactc tccagtctc                                                    19



<210>  20
<211>  19
<212>  DNA
<213>  Artificial sequence

<220>
<223>  GRM5 reverse primer

<400>  20
tcacaacgat gaagaactc                                                    19



<210>  21
<211>  24
<212>  DNA
<213>  Artificial sequence

<220>
<223>  GRM6 forward primer

<400>  21
ccaaaccacc acactaactg tgtc                                              24



<210>  22
<211>  22
<212>  DNA

<213>  Artificial sequence

<220>
<223>  GRM6 reverse primer

<400>  22
ggatggaaca ggatgacgta gg                                                22

<210>  23
<211>  19
<212>  DNA
<213>  Artificial sequence

<220>
<223>  GRM7 forward primer

<400>  23
tctaacctgt tccatgcca                                                    19

<210>  24
<211>  18
<212>  DNA
<213>  Artificial sequence

<220>
<223>  GRM7 reverse primer

<400>  24
ataggaagcg ggcaggac                                                     18

<210>  25
<211>  22
<212>  DNA
<213>  Artificial sequence

<220>
<223>  GRM8 forward primer

<400>  25
cgcaagagaa gcttcaaggc tg                                                22

<210> 26
<211> 22
<212> DNA
<213> Artificial sequence

<220>
<223> GRM8 reverse primer

<400> 26
tcacctcgcc atttggtctg tc 22